# FASCICULE DE BREVET EUROPEEN

(11) **EP 2 820 055 B1**
(45) Date de publication et mention de la délivrance du brevet: **14.09.2016**
(21) Numéro de dépôt: 13710468.3
(22) Date de dépôt: 15.02.2013
(51) Int. Cl.: C08F 230/08, C08F 220/22, C08F 220/38, C08F 222/24, A61K 8/84, A61K 47/34, A61Q 1/00, A61Q 19/00, C08F 283/12, A61Q 5/00

(54) **NOUVEAU POLYMÈRE D'ACRYLATE DE SILICONE ET DE MÉTHACRYLATE DE TRIFLUORO-ÉTHYLE, PRÉPARATION ET UTILISATION EN COSMÉTIQUE**
NEUES SILIKONACRYLAT- UND TRIFLUORETHYLMETHACRYLATPOLYMER, HERSTELLUNG UND VERWENDUNG DAVON IN KOSMETIKA
NEW SILICONE ACRYLATE AND TRIFLUOROETHYL METHACRYLATE POLYMER, PREPARATION AND USE THEREOF IN COSMETICS

(30) Priorité: 29.02.2012 FR 1251848
(43) Date de publication de la demande: 07.01.2015
(73) Titulaire: Societe D Exploitation De Produits Pour Les Industries Chimiques Seppic, 75007 Paris (FR)
(72) Inventeur: BRAUN, Olivier, F-42170 St Just St Rambert (FR); MALLO, Paul, F-78110 Le Vésinet (FR)
(74) Mandataire: Conan, Philippe Claude
(86) Numéro de dépôt international: PCT/FR2013/050319
(87) Numéro de publication internationale: WO 2013/128095

(56) Documents cités:
- EP-A1- 0 412 771
- WO-A2-02/40603
- US-A1- 2011 123 933

## Description

L'invention a pour objet de nouveaux agents épaississants ainsi que leur utilisation en cosmétique et en pharmacie.

Il est bien connu d'épaissir des phases aqueuses destinées à des applications cosmétiques, dermo-pharmaceutiques ou pharmaceutiques, en y introduisant des polymères hydrophiles synthétiques ou naturels. Les polymères naturels tels que les gommes de xanthane ou de guar sont assez largement utilisés mais présentent les inconvénients classiques des produits naturels (qualité et prix fluctuants). C'est pourquoi les polymères épaississants synthétiques sont largement utilisés pour augmenter la viscosité des crèmes, des émulsions et de diverses solutions topiques. Ils se présentent soit sous forme de poudre, soit sous forme liquide. Selon cette dernière option, le polymère est préparé par polymérisation en émulsion inverse à l'aide d'agents tensioactifs et la forme liquide résultante est une émulsion eau dans huile contenant le polymère, émulsion que l'on appelle couramment latex inverse.

Les polymères épaississants sous forme de poudre, les plus connus sont les polymères à base d'acide acrylique ou les copolymères à base d'acide acrylique et de ses esters, comme les polymères commercialisés sous les noms, CARBOPOL™ et PEMULEN™ et qui sont décrits notamment dans les brevets américains US 5, 373,044, US 2,798,053 ainsi que dans le brevet européen EP 0 301 532, ou encore comme les homopolymères ou copolymères à base d'acide 2-acrylamido-2-méthyl-propane sulfonique commercialisés sous le nom ARISTOFLEX™ et qui sont décrits notamment dans les brevets européens EP 0 816 403, EP 1 116 733 et EP 1 069 142. Ces poudres de polymère sont obtenues par polymérisation précipitante dans un solvant organique comme le benzène, l'acétate d'éthyle, le cyclohexane, ou le tertio-butanol.

Les latex inverses, comme ceux commercialisés sous les noms SEPIGEL™ 305, SIMULGEL™ 600, SIMULGEL™ EG, SIMULGEL™ EPG, SIMULGEL™ NS, SIMULGEL™ INS 100 SIMULGEL™ A, SEPIPLUS™ 400, SEPIPLUS™ 250, SEPIPLUS™ 265 et SEPIPLUS™ S, obtenus par polymérisation en émulsion inverse, sont plus aisément manipulables et se dispersent très rapidement dans l'eau. Ils développent des performances épaississantes remarquablement élevées qui sont probablement la conséquence du procédé pour leur préparation, une réaction de polymérisation en phase dispersée, qui conduit à des polymères de très hauts poids moléculaires.

Les polymères susmentionnés sont essentiellement destinés à épaissir des phases aqueuses des formulations topiques cosmétiques, dermo-pharmaceutiques ou pharmaceutiques.

Or certaines formulations, plus particulièrement celles destinées au soin de la peau, contiennent aussi des quantités relativement importantes de glycérine (ou glycérol), typiquement entre 5% et 10% massique, pour augmenter leur potentiel hydratant. Mais comme la présence de glycérine en leur sein augmente aussi considérablement leur effet collant, les préparateurs y ajoutent des huiles de silicone pour limiter ou annihiler cet effet collant.

Cependant l'addition d'huiles de silicone complexifie la préparation de ces formules. De plus, la présence d'huiles de silicone dans des formules, qui sont destinées à être en contact direct avec la peau, est mal perçue par le consommateur final. L"industrie cosmétique essaye donc d'en limiter l'utilisation.

Les inventeurs ont donc cherché à développer de nouveaux polymères épaississants qui soient efficaces sur une large gamme de pH, et qui soient capables de réduire ou d'annihiler l'effet collant induit par la présence de glycérine, sans qu'il soit nécessaire d'ajouter un tiers composés comme les dérivés siliconés. Ils ont trouvé que les poudres de polymères issus de la polymérisation précipitante de monomères fluorés et de monomère possédant une fonction acide fort, résolvaient ces problèmes.

C'est pourquoi selon un premier aspect, l'invention a pour objet un polyélectrolyte anionique linéaire, branché ou réticulé, issu de la polymérisation pour 100% massique :
a) d'une proportion massique supérieure ou égale à 70% et inférieure ou égale à 98,5% d'unités monomériques issues d'un monomère comportant une fonction acide fort, libre partiellement ou totalement salifiée ;
b) d'une proportion massique supérieure ou égale à 0,5% et inférieure ou égale à 10%,
   - Soit d'un monomère de formule (Ia) :

      R-(CH₂)₃-Si(CH₃)₂-[O-Si(CH₃)₂-]ₙO-Si(CH₃)₂-(CH₂)₃-R (la),

      dans laquelle :
      R représente le radical monovalent :

         -(O-CH₂-CH₂-)ₓ[O-CH₂-CH(CH₃)-]_{y}O-C(=O)-CH=CH₂,

         dans lequel x et y représentent, indépendant l'un de l'autre, un nombre entier supérieur ou égal à 0 et inférieur ou égal à 40, étant entendu que la somme x + y est supérieure à 0 et inférieure à 50 ; et
      n représente un nombre entier supérieur ou égal à 0 et inférieur ou égal à 45, ledit monomère de formule (la) ayant un poids moléculaire supérieur ou égal à 1.500 et inférieur ou égal à 7.000 ;
   - Soit d'un monomère de formule (Ib) :

      Si(CH₃)₃-[O-Si(CH₃)₂-]ₘ[O-Si(CH₃)[(CH₂)₃-R]-]ₚO-Si(CH₂)₃ (Ib),

      dans laquelle :
      R représente un radical monovalent de formule :

         -(O-CH₂-CH₂-)ₓ[O-CH₂-CH(CH₃)-]_{y}O-C(=O)-CH=CH₂,

         dans lequel x et y représentent, indépendant l'un de l'autre, un nombre entier supérieur ou égal à 0 et inférieur ou égal à 40, étant entendu que la somme x + y est supérieure à 0 et inférieure à 50 ;
      m représente un nombre entier supérieur ou égal à 1 et inférieur ou égal à 7, et
      p représente indépendamment de m un nombre entier supérieur ou égal à 1 et inférieur à 20 ;
      ledit monomère de formule (Ib) ayant un poids moléculaire supérieur ou égal à 1.000 et inférieur ou égal à 4.000 ;
c) d'une proportion massique supérieure ou égale à 1% et inférieure ou égale à 20% d'un monomère de formule (II) :

   CH₂=CH(R₁)-C(=O)-O-(CH₂)ₙ-CF₃ (II),

   formule (II) dans laquelle le radical R₁ représente un atome d'hydrogène ou un radical méthyle, et n représente un nombre entier égal à 1, 2 ou 3 ;
d) optionnellement d'une proportion massique supérieure à 0% et inférieure ou égale à 5%, d'unités monomériques issues d'un monomère de formule (III) :

   R₂-C(=O)-O-[(CH₂-CH(R₄)-O]ₘ-R₃ (III),

   formule (III) dans laquelle m représente un nombre supérieur ou égal à 0 et inférieur ou égal à 50, R₂ représente un radical monovalent aliphatique insaturé comprenant de 2 à 4 atomes de carbone, R₄ représente un atome d'hydrogène, un radical méthyle ou un radical éthyle et R₃ représente un radical aliphatique hydrocarboné, linéaire ou ramifié, saturé ou insaturé, comprenant de 8 à 30 atomes de carbone, et
e) optionnellement d'une proportion massique supérieure à 0% et inférieure ou égale à 5% d'au moins un monomère de réticulation diéthylénique ou polyéthylénique.

Par polyélectrolyte branché, on désigne un polyélectrolyte non linéaire qui possède des chaînes pendantes de manière à obtenir, lorsqu'il est mis en solution dans l'eau, un fort état d'enchevêtrement conduisant à des viscosités, à bas gradient de vitesse, très importantes.

Par polyélectrolyte réticulé, on désigne un polyélectrolyte non linéaire se présentant à l'état de réseau tridimensionnel insoluble dans l'eau, mais gonflable à l'eau et conduisant donc à l'obtention d'un gel chimique.

Le polyélectrolyte obtenu par le procédé selon l'invention peut comporter des motifs réticulés et/ou des motifs branchés.

Selon un aspect particulier de la présente invention, dans la formule (II) telle que définie ci-dessus, n est égal à 1.

Selon un autre aspect particulier de la présente invention, dans la formule (II) telle que définie ci-dessus, R₁ représente un radical méthyle.

Par monomère comportant une fonction acide fort libre partiellement ou totalement salifiée, on désigne notamment les monomères possédant une fonction sulfonique (-SO₃H).

Selon un aspect particulier, ledit monomère comportant une fonction acide fort libre partiellement ou totalement salifiée, est l'acide 2-méthyl 2-[(1-oxo 2-propènyl) amino] 1-propanesulfonique libre, partiellement salifié ou totalement salifié.

Par fonction acide fort partiellement ou totalement salifiée, on désigne dans le cadre de la présente invention, une fonction acide partiellement ou totalement salifiée notamment sous forme d'un sel de métal alcalin, tel que par exemple le sel de sodium ou le sel de potassium ou sous forme d'un sel d'ammonium.

Selon un autre aspect particulier, l'invention a pour objet un polyélectrolyte anionique tel que défini précédemment, dans lequel ledit au moins un monomère comportant une fonction acide fort partiellement ou totalement salifiée, est l'acide 2-méthyl 2-[(1-oxo 2-propènyl) amino] 1-propanesulfonique, partiellement ou totalement salifié sous forme de sel d'ammonium.

Selon un autre aspect particulier, le polyélectrolyte linéaire, branché ou réticulé, est caractérisé en ce que la proportion massique en unités monomériques issues du monomère comportant une fonction acide fort libre partiellement ou totalement salifiée, est inférieure ou égale à 95%.

Dans la formule (III) telle que définie précédemment, le radical divalent :

-[(CH₂-CH(R₄)-O]ₘ-

représente notamment :
- Soit une chaîne composée uniquement de groupes éthoxyle (R₄ = H ; n >0),
- Soit une chaîne composée uniquement de groupes propoxyle (R₄ = CH₃ n > 0),
- Soit une chaîne composée uniquement de groupes butoxyle (R₄ = C₂H₅ ; n> 0),
- Soit une chaîne composée d'au moins deux groupes différents choisis parmi les groupes éthoxyle propoxyle et/ou butoxyle.

Lorsque cette chaîne est composée de groupes différents, ils sont distribués tout au long de cette chaîne, de façon séquencée ou aléatoire.

Par radical aliphatique, hydrocarboné linéaire, saturé ou insaturé, comprenant de 8 à 30 atomes de carbone, on désigne plus particulièrement pour R₃, dans la formule (II) telle que définie précédemment :
- Soit un radical dérivé des alcools primaires linéaires tels que par exemple, ceux dérivés des alcools octylique, pélargonique, décylique, undécylique, undécénylique, laurique, tridécylique, myristylique, pentadécylique, cétylique, heptadécylique, stéarylique, oléylique, linoléylique, nonadécylique, arachidique, béhènylique, érucylique ou I-triacontanoïque. Il s'agit alors des radicaux octyle, nonyle, décyle, undécyle, 10-undécènyle, dodécyle, tridécyle, tétradécyle, pentadécyle, hexadécyle, heptadécyle, octadécyle, 9-octadécènyle, 10,12-octadécadiènyle, 13-docosènyle ou triacontanyle ;
- Soit un radical dérivé des alcools de Guerbet, qui sont des 1-alcanols ramifiés répondant à la formule générale :

   CH₃-(CH₂)ₚ-CH[CH₃-(CH₂)ₚ₋₂]-CH₂OH,

   dans laquelle p représente un nombre entier compris entre 2 et 14, tels que, par exemple, les radicaux 2-éthyl hexyle, 2-propyl heptyle, 2-butyl octyle, 2-pentyl nonyle, 2-hexyl décyle ou 2-octyl dodécyle ;
- Soit un radical dérivé des isoalcanols répondant à la formule générale :

   CH₃-CH(CH₃)-(CH₂)ₘ-CH₂OH,

   dans laquelle m représente un nombre entier compris entre 2 et 26 tels que, par exemple, les radicaux 4-méthyl pentyle, 5-méthyl hexyle, 6-méthyl heptyle, 15-méthyl pendadécyle ou 16-méthyl heptadécyle ;
- Soit les radicaux 2-hexyl octyle, 2-octyl décyle ou 2-hexyl dodécyle.

Par radical aliphatique hydrocarboné, linéaire ou ramifié, saturé ou insaturé, comprenant de 8 à 30 atomes de carbone, on désigne plus particulièrement pour R₃; dans la formule (III) telle que définie précédemment, un radical alkyle comportant de 12 à 22 atomes de carbone.

Dans la formule (III) telle que définie précédemment, m représente plus particulièrement un nombre supérieur ou égal à 0 et inférieur ou égal à 25.

Dans la formule (III) telle que définie précédemment, R₂ représente plus particulièrement le radical vinyle (CH₂=CH-) ou le radical isopropènyle [CH₂=C(CH₃)-].

Selon un aspect plus particulier de la présente invention, ledit monomère de formule (III) telle que définie précédemment, est choisi parmi :
- Le méthacrylate de béhènyle pentacosaéthoxylé, composé de formule (III) telle que définie précédemment, dans laquelle R₃ représente le radical docosanyle, R₂ représente le radical isopropènyle, R₄ représente un atome d'hydrogène et n est égal à 25;
- L'acrylate de lauryle tétraéthoxylé, composé qui correspond à la formule (III) telle que définie précédemment, dans laquelle R₃ représente le radical dodécyle, R₂ représente le radical vinyle, R₄ représente un atome d'hydrogène et n est égal à 4,
- Le méthacrylate de stéaryle eicosaéthoxylé, composé de formule (III) telle que définie précédemment, dans laquelle R₃ représente le radical stéaryle, R₂ représente le radical isopropènyle, R₄ représente un atome d'hydrogène et n est égal à 20,
- Le méthacrylate de lauryle tétraéthoxylé, composé qui correspond à la formule (III) telle que définie précédemment, dans laquelle R₃ représente le radical dodécyle, R₂ représente le radical isoprènyle, R₄ représente un atome d'hydrogène et n est égal à 4, ou
- Le méthacrylate de stéaryle, composé de formule (III) telle que définie précédemment, dans laquelle R₃ représente le radical stéaryle, R₂ représente le radical isopropènyle, R₄ représente un atome d'hydrogène et n est égal à 0.

Selon un aspect plus particulier de la présente invention, ledit monomère de formule (la) telle que définie précédemment, est le SILMER™ ACR Di-50 commercialisé par la société SILTECH, de poids moléculaire égal à 4.100 et référencé dans le "Chemical Abstracts sous le numéro de registre RN = 128754-61-6.

Selon un aspect plus particulier de la présente invention, ledit monomère de formule (la) telle que définie précédemment, est le SILMER™ ACR Di-10 commercialisé par la société SILTECH, de poids moléculaire égal à 1.100.

Selon un aspect plus particulier de la présente invention, ledit monomère de formule (Ib) telle que définie précédemment, est le SILMER™ ACR D208 commercialisé par la société SILTECH, de poids moléculaire égal à 3.000 et référencé dans le "Chemical Abstracts" sous le numéro de registre RN= 518299-28-0.

Selon un autre aspect plus particulier de la présente invention, ledit monomère de formule (Ib) telle que définie précédemment, est le SILMER™ ACR D2 commercialisé par la société SILTECH, de poids moléculaire égal à 1.400 et référencé dans le "Chemical Abstracts" sous le numéro de registre RN = 158061-40-6.

Selon un aspect particulier, l'invention a plus particulièrement pour objet un polyélectrolyte tel que défini précédemment, issu de la polymérisation pour 100% massique :
a) d'une proportion massique supérieure ou égale à 75% et inférieure ou égale à 90% d'unités monomériques issues d'un monomère comportant une fonction acide fort, libre, partiellement ou totalement salifiée,
b) d'une proportion massique supérieure ou égale à 1% et inférieure ou égale à 10% d'unités monomériques issues d'un monomère de formule (la) ou d'un monomère de formule (Ib) telles que définies précédemment ;
c) d'une proportion massique supérieure ou égale à 5% et inférieure ou égale à 20% d'unités monomériques issues du méthacrylate de 2,2,2-trifluoro éthyle ; et
d) optionnellement d'une proportion massique supérieure à 0% et inférieure ou égale à 5% d'unités monomériques issues du composé de formule (III) telle que définie précédemment.

Selon un autre aspect particulier de la présente invention, le polyélectrolyte tel que défini ci-dessus est réticulé.

Selon ce dernier aspect, ledit au moins un monomère de réticulation diéthylénique ou polyéthylénique est choisi notamment parmi l'acide diallyloxyacétique ou un des sels comme son sel de sodium, le triallylamine, le triacrylate de triméthylol propane, le diméthacrylate d'éthylèneglycol, le diacrylate de diéthylène glycol, le diallylurée ou le méthylène bis(acrylamide) ou un mélange de plusieurs de ces composés.
selon un aspect tout particulier de la présente invention, l'agent réticulant mis en oeuvre est le méthylène bis(acrylamide) ou le triacrylate de triméthylol propane (TMPTA).

L'agent de réticulation est alors généralement mis en oeuvre dans la proportion massique, exprimée par rapport aux monomères mis en oeuvre, de 0,005% massique à 5% massique, et plus particulièrement de 0,5% massique à 2,5% massique.

Selon un aspect particulier, l'invention a plus particulièrement pour objet un polyélectrolyte tel que défini précédemment, issu de la polymérisation pour 100% massique :
a) d'une proportion massique supérieure ou égale à 75% et inférieure ou égale à 85% d'unités monomériques issues d'un monomère comportant une fonction acide fort, libre, partiellement ou totalement salifiée ;
b) d'une proportion massique supérieure ou égale à 1% et inférieure ou égale à 10% d'unités monomériques issues d'un monomère de formule (la) ou d'un monomère de formule (Ib) telles que définies précédemment ;
c) d'une proportion massique supérieure ou égale à 5% et inférieure ou égale à 20% d'unités monomériques issues du méthacrylate de 2,2,2-trifluoro éthyle ; et
e) d'une proportion massique supérieure à 0% et inférieure ou égale à 5% massique d'unités monomériques issues d'au moins un monomère de réticulation diéthylénique ou polyéthylénique.

L'invention a aussi pour objet un procédé de préparation du polyélectrolyte tel que défini précédemment, caractérisé en ce qu'il comprend:
Une étape a) de préparation d'un mélange réactionnel comprenant dans les proportions molaires souhaitées et dans un solvant (S), le ou les monomères comportant une fonction acide fort, libre, partiellement ou totalement salifiée, le monomère de formule (la) ou le monomère de formule (Ib) le monomère de formule (II) ; si nécessaire ou si désiré les unités monomériques issues du composé de formule (III) ; et si nécessaire ou si désiré le ou les monomères de réticulation diéthyléniques ou polyéthyléniques, ledit solvant (S) étant :
   - Soit une cétone de formule (IV) : 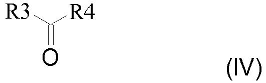 dans laquelle R3 et R4 identiques ou différents, représentent indépendamment l'un de l'autre un radical méthyle, un radical éthyle, un radical isopropyle ou un radical isobutyle ;
   - Soit un mélange consistant en, pour 100% molaire :
      - de l'eau en une proportion supérieure à 0% molaire et inférieure ou égale à 25% molaire ; et
      - une cétone de formule (IV) telle que définie ci-dessus, en une proportion supérieure ou égal à 75% molaire et inférieure à 100% ;
   - Soit le tertio-butanol ;

Une étape b) au cours de laquelle la réaction de polymérisation est amorcée par introduction dans ledit mélange réactionnel préparé à l'étape a), d'un initiateur de radicaux libres, puis est laissée se dérouler jusqu'à sa conclusion, pour obtenir un précipité dudit polyélectrolyte.

Selon un autre aspect particulier de la présente invention, dans l'étape b) du procédé tel que défini précédemment, la réaction de polymérisation est amorcée à une température égale ou supérieure à 50°C à l'aide d'un amorceur radicalaire produisant des radicaux par homolyse, tel que le peroxyde de dilauroyle, l'azo-bis(isobutyronitrile) ou encore les dérivés azoïques.

Selon un autre aspect particulier de la présente invention, dans l'étape b) du procédé tel que défini précédemment, la réaction de polymérisation est amorcée par un couple oxydoréducteur tel qu'un couple oxydoréducteur générateur d'ions hydrogénosulfite (HSO₃) comme le couple hydroperoxyde de cumène - métabisulfite de sodium (Na₂S₂O₅) ou le couple hydroperoxyde de cumène-chlorure de thionyle (SOCl₂) à une température inférieure ou égale à 20°C, si désiré accompagné d'un agent co-initiateur de polymérisation tel que par exemple l'azo-bis(isobutyronitrile), le dilaurylperoxyde ou le persulfate de sodium, puis conduite de manière quasi-adiabatique.

Le procédé tel que défini ci-dessus peut comprendre en outre :
une étape c) d'isolation dudit précipité obtenu à l'étape b) par séparation d'avec ledit solvant (S), puis si nécessaire ou si désiré,
une étape d) de séchage dudit précipité résultant de l'étape c).

Selon un autre aspect particulier de la présente invention, dans l'étape c) du procédé tel que défini précédemment, la séparation du précipité obtenu dudit solvant organique est effectué par filtration.

Selon un autre aspect particulier, l'invention a pour objet un procédé tel que défini ci-dessus, dans lequel ledit solvant (S) est :
Soit de l'acétone, soit un mélange eau-acétone en un ratio molaire eau/acétone supérieur à 0 et inférieur ou égal à 5/95, soit du tertio-butanol.

L'invention a aussi pour objet l'utilisation du polyélectrolyte anionique tel que défini précédemment, comme épaississant et/ou comme stabilisant et/ou comme émulsionnant, d'une composition topique cosmétique, dermopharmaceutique ou pharmaceutique.

Une composition topique selon l'invention, destinée à être appliquée sur la peau ou les muqueuses de l'homme ou de l'animal, peut consister en une émulsion topique comprenant au moins une phase aqueuse et au moins une phase huile. Cette émulsion topique peut être du type huile-dans-eau (H/E), eau-dans-huile (E/H), huile-dans-eau-dans-huile (H/E/H) ou eau-dans-huile-dans-eau (E/H/E). La phase huile de l'émulsion topique peut consister en un mélange d'une ou plusieurs huiles.

Une composition topique selon l'invention peut être destinée à une utilisation cosmétique ou être utilisée pour préparer un médicament destiné au traitement des maladies de la peau, du cuir chevelu et des muqueuses. Dans ce dernier cas, la composition topique comporte alors un principe actif qui peut par exemple, consister en un agent anti-inflammatoire, un myorelaxant, un antifongique, un antibactérien ou antipelliculaire.

Lorsque la composition topique est utilisée en tant que composition cosmétique destinée à être appliquée sur la peau, sur le cuir chevelu ou les muqueuses, elle peut ou non comporter un principe actif, par exemple un agent hydratant, un agent bronzant, un filtre solaire, un antirides, un agent à visée amincissante, un agent antiradicalaire, un agent anti-acnéique, un antifongique ou antipelliculaire.

La composition topique selon l'invention comporte habituellement entre 0,1 % et 10 % massique et plus particulièrement de 1 % à 5 % massique du polyélectrolyte anionique tel que défini précédemment.

Selon un aspect particulier La composition topique telle que définie ci-dessus comprend en outre de 1% massique à 10% massique de glycérol.

Le pH de la composition topique est de préférence supérieur ou égal à 3.

La composition topique peut en outre comporter des composés classiquement compris dans ce type de compositions, par exemple des parfums, des conservateurs, des colorants, des pigments, des écrans solaires, des ingrédients actifs, des émollients ou des tensioactifs.

Le polyélectrolyte anionique selon l'invention est un substitut intéressant aux latex inverses vendus sous les noms de SEPIGEL™ 305, SEPIGEL™ 501, SIMULGEL™ EG, SIMULGEL™ EPG, SIMULGEL™ NS, SIMULGEL™ INS 100, SIMULGEL™ 600, SIMULGEL™ A, SEPIPLUS™ 265, SEPIPLUS™250, SEPIPLUS™ 400 ou SEPIPLUS™ S par la demanderesse, car il présente aussi une bonne compatibilité avec les autres excipients utilisés pour la préparation de formulations telles que les laits, les lotions, les crèmes, les savons, les bains, les baumes, les shampooings ou les après-shampooings. Il peut aussi être mis en oeuvre avec lesdits SEPIGEL™ et/ou SIMULGEL™ et/ou SEPIPLUS™.

Il est notamment compatible avec les concentrés décrits et revendiqués dans les publications internationales WO 92/06778, WO 95/04592, WO 95/13863, WO 96/37285, WO 98/22207, WO 98/47610 ou dans FR 2 734 496, avec les agents tensioactifs décrits dans WO 93/08204. Il est particulièrement compatible avec le MONTANOV™ 68, le MONTANOV™ 82, le MONTANOV™ 202, le MONTANOV™ L, le MONTANOV™ S, le FLUIDANOV™ 20X ou l'EASYNOV™.

Il peut également être utilisé pour former des gels aqueux à pH acide cosmétiquement ou physiologiquement acceptables, tels que ceux décrit dans WO 93/07856; elle peut également être utilisée en association avec des celluloses non-ioniques, pour former par exemple des gels de coiffage tels que ceux décrits dans EP 0 684 024, ou encore en association avec des esters d'acides gras et de sucre, pour former des compositions pour le traitement du cheveu ou de la peau telles que celles décrites dans EP 0 603 019, ou encore dans les shampooings ou après-shampooings tels que décrits et revendiqués dans WO 92/21316 ou enfin en association avec un homopolymère anionique tels que le CARBOPOL™ pour former des produits de traitement des cheveux comme ceux décrits dans DE 19523596.

Il est également compatible avec les dérivés N-acylés d'aminoacides, ce qui permet son utilisation dans des compositions apaisantes notamment pour peau sensible, telles que celles décrites ou revendiquées dans WO 92/21318, WO 94/27561 ou WO 98/09611. Il est également compatible avec des polymères épaississants et/ou gélifiants comme les hydrocolloïdes d'origine végétale ou biosynthétique, par exemple la gomme xanthane, la gomme de karaya, les carraghénates, les alginates, les galactomannanes ; comme les silicates ; comme la cellulose et ses dérivés ; comme l'amidon et ses dérivés hydrophiles ; comme les polyuréthanes.

Le polyélectrolyte anionique selon l'invention permet de plus de s'affranchir de l'utilisation d'huile de silicone dans les compositions topiques comprenant du glycérol, en ce qu'elle inhibe l'effet collant induit par ce triol.

C'est pourquoi, selon un dernier aspect, l'invention a pour objet une composition topique comprenant entre 0,1 % et 10 % massique et plus particulièrement de 1 % à 5 % massique du polyélectrolyte anionique tel que défini précédemment et de 1% massique à 10% massique de glycérol et caractérisée en ce qu'elle est exempte d'huile de silicone

Les exemples suivants illustrent l'invention sans toutefois la limiter.

### Exemple 1: Préparation d'un copolymère ATBS/MATRIFE/ACR-D2 réticulé au TMPTA

On charge dans un réacteur maintenu à 25°C sous agitation et contenant 487,5g de tertio-butanol, 67,7g d'acide 2-méthyl 2-[(1-oxo 2-propènyl) amino] 1-propanesulfonique (ATBS), puis on y ajoute de l'ammoniac jusqu'à atteindre une valeur de pH environ de 6. Après un temps suffisant pour atteindre une bonne homogénéisation de la solution, celle-ci est désoxygénée par barbotage d'azote, puis on ajoute 13,8g de méthacrylate de (2,2,2-trifluoro éthyle) (MATRIFE), 4,3g de SILMER™ ACR D2 (composé de formule (Ib), identifié sous le numéro Chemical Abstract RN= 158061-40-6, de poids moléculaire égal à 1.400), 12,5g d'eau permutée et 2g de triacrylate de triméthylolpropane (TMPTA).

On laisse le mélange réactionnel sous agitation pendant soixante minutes; puis on le chauffe jusqu'à atteindre la température de 60°C. On y ajoute alors 1 g de peroxyde de dilauroyle. Le milieu réactionnel est ensuite de nouveau laissé sous agitation pendant environ 60 minutes puis porté à 80°C et laissé à cette température pendant soixante minutes. On ajoute 0,33 g supplémentaire de peroxyde de dilaurolyle et on maintient le milieu sous agitation à 80°C pendant deux heures.

Après refroidissement, la poudre qui s'est formée en cours de polymérisation, est filtrée et séchée pour obtenir le produit désiré, dénommé par la suite : Polyélectrolyte 1.

### Evaluation du pouvoir épaississant

Viscosité (µ) d'une dispersion aqueuse à 2% massique de Polyélectrolyte 1 [Brookfield RVT, Mobile 6, Vitesse : 5 tours/minute (M6, V5)] : **µ = 102.000 mPas.**

Viscosité (µ) d'une dispersion aqueuse à 2% massique de Polyélectrolyte 1 et 1% massique de chlorure de sodium [Brookfield RVT, (M6, V5)] : **µ = 2.900 mPas.**

### Evaluation du caractère collant de gels aqueux contenant de la glycérine

On prépare 250g d'un gel aqueux en mélangeant 2,5 g du Polyélectrolyte 1, 25g de glycérine et 222,5g d'eau.

A titre de base de comparaison on prépare 250g d'un gel aqueux en mélangeant 6,25g de SEPIGEL™ 305, 25 g de glycérine et 218,75g d'eau.

En étalant chacun des deux gels sur une surface différente du dessus de la main, on observe l'absence d'effet collant pour le gel comprenant le Polyélectrolyte 1 selon l'invention contrairement au gel selon l'état de la technique.

### Exemple 2: Préparation d'un copolymère ATBS/MATRIFE/ACR-D2/MAS réticulé au TMPTA

On opère de la même façon qu'à l'exemple 1 mais en utilisant 2,6g de SILMER™ ACR D2 et en réduisant la teneur en TMPTA à 1,2 g. 1,4 g de méthacrylate de stéaryle (MAS) sont également ajoutés dans le milieu réactionnel avant polymérisation. On obtient le Polyélectrolyte 2.

### Evaluation du pouvoir épaississant

Viscosité (µ) d'une dispersion aqueuse à 1% massique de Polyélectrolyte 2 [Brookfield RVT, (M6,V5)] : **µ= 67.600 mPas.**

Viscosité **(µ)** d'une dispersion aqueuse à 1% massique de polyélectrolyte 2 et 1‰ massique de chlorure de sodium [Brookfield RVT, (M6,V5)] : **µ = 12.660 mPas.**

### Evaluation du caractère collant de gels aqueux contenant de la glycérine

On prépare 250g d'un gel aqueux en mélangeant 2,5 g du Polyélectrolyte 2, 25g de glycérine et 222,5g d'eau.

En étalant chacun ce gel sur le dessus de la main, on observe l'absence d'effet collant pour le gel comprenant le Polyélectrolyte 2 selon l'invention.

### Exemple 3: Préparation d'un copolymère ATBS/MATRIFE/ACR-Di-50/MAS réticulé au TMPTA

On opère de la même façon qu'à l'exemple 2 mais en utilisant 2,6g de SILMER™ ACR Di-50 (composé de formule (la), identifié sous le numéro Chemical Abstract RN= 128754-61-0, de poids moléculaire égal à 4100) à la place du SILMER™ ACR D2. On obtient le Polyélectrolyte 3.

### Evaluation du pouvoir épaississant

Viscosité (µ) d'une dispersion aqueuse à 1% massique de Polyélectrolyte 3 [Brookfield RVT, (M6,V5)] : **µ = 39.200 mPas.**

Viscosité (µ) d'une dispersion aqueuse à 1% massique de polyélectrolyte 3 et 1‰ massique de chlorure de sodium [Brookfield RVT, (M6,V5)] : **µ = 10.940 mPas.**

### Evaluation du caractère collant de gels aqueux contenant de la glycérine

On prépare 250g d'un gel aqueux en mélangeant 2,5 g du Polyélectrolyte 3, 25g de glycérine et 222,5g d'eau.

En étalant chacun ce gel sur le dessus de la main, on observe l'absence d'effet collant pour le gel comprenant le Polyélectrolyte 3 selon l'invention.

### Exemple 4: Préparation d'un copolymère ATBS/MATRIFE/ACR-D2

On opère de la même façon qu'à l'exemple 1 mais en utilisant 7,1 g de SILMER™ ACR D2 et en retirant le TMPTA. On obtient le Polyélectrolyte 4.

### Evaluation du pouvoir épaississant

Viscosité (µ**)** d'une dispersion aqueuse à 1% massique de Polyélectrolyte 4 [Brookfield RVT, (M6,V5)] : **µ = 45.000 mPas.**

Viscosité (µ) d'une dispersion aqueuse à 1% massique de polyélectrolyte 4 et 1‰ massique de chlorure de sodium [Brookfield RVT, (M6,V5)] : **µ = 2.820 mPas.**

### Evaluation du caractère collant de gels aqueux contenant de la glycérine

On prépare 250g d'un gel aqueux en mélangeant 2,5 g du Polyélectrolyte 4, 25g de glycérine et 222,5g d'eau.

En étalant chacun ce gel sur le dessus de la main, on observe l'absence d'effet collant pour le gel comprenant le Polyélectrolyte 4 selon l'invention.

### Exemples de formulations comprenant de la glycérine et exemptes d'huiles de silicone préparées avec des polyélectrolytes selon l'invention

### Exemple 5 : Emulsion démaquillante à l'huile d'amandes douces

| | |
|---|---|
| MONTANOV™ 68 : | 5% |
| Huile d'amandes douces : | 5% |
| Eau : | q.s.p. 100% |
| Polyélectrolyte 1 : | 0,3% |
| Glycérine : | 5% |
| Conservateur : | 0,2% |
| Parfum : | 0,3% |

### Exemple 6 : Emulsion pour peaux à tendance atopique

| | |
|---|---|
| ARLACEL™ P135 : | 2,00% |
| Polyélectrolyte 1 : | 1,00% |
| LANOL™ 1688 : | 14,00% |
| PRIMOL™ 352 : | 8,00% |
| Glycérine : | 5,00% |
| Eau: | qsp 100% |
| Sulfate de magnésium : | 0,70% |
| SEPICIDE™ HB : | 0,30% |
| SEPICIDE™ Cl : | 0,20% |
| MICROPEARL™ M310 : | 5,00% |

Les définitions des produits commerciaux utilisés dans les exemples, sont les suivantes :
Le MONTANOV™ 68 (cétéaryl glucoside), est une composition auto-émulsionnable telle que décrite dans WO 92/06778, commercialisée par la société SEPPIC.
L'ARLACEL™ P135 est un mélange de monostéarate de glycérol, de distéarate de glycérol et de polyoxyéthylène-stéarate de glycérol commercialisé par la société CRODA.
Le SEPICIDE™ Cl, imidazolidine urée, est un agent conservateur commercialisé par la société SEPPIC.
Le LANOL™ 1688 est un ester émollient à effet non gras commercialisé par la société SEPPIC.
Le SEPICIDE™ HB, qui est un mélange de phénoxyéthanol, de méthylparaben, d'éthylparaben, de propylparaben et de butylparaben, est un agent conservateur commercialisé par la société SEPPIC.
Le MICROPEARL™ M 310 est une poudre ultra fine au toucher très doux et à action matifiante commercialisée par la société MATSUMO.
Le PRIMOL™ 352 est une huile minérale commercialisée par la société EXXON.

## Revendications

1. Polyélectrolyte anionique linéaire, branché ou réticulé, issu de la polymérisation pour 100% massique :
a) d'une proportion massique supérieure ou égale à 70% et inférieure ou égale à 98,5% d'unités monomériques issues d'un monomère comportant une fonction acide fort, libre partiellement ou totalement salifiée ;
b) d'une proportion massique supérieure ou égale à 0,5% et inférieure ou égale à 10%,
- Soit d'un monomère de formule (Ia) :
R-(CH₂)₃-Si(CH₃)₂-[O-Si(CH₃)₂-]ₙO-Si(CH₃)₂-(CH₂)₃-R (Ia),
dans laquelle :
R représente le radical monovalent :
-(O-CH₂-CH₂-)ₓ[O-CH₂-CH(CH₃)-]_{y}O-C(=O)-CH=CH₂,
dans lequel x et y représentent, indépendant l'un de l'autre, un nombre entier supérieur ou égal à 0 et inférieur ou égal à 40, étant entendu que la somme x + y est supérieure à 0 et inférieure à 50 ; et
n représente un nombre entier supérieur ou égal à 0 et inférieur ou égal à 45,
ledit monomères de formule (la) ayant un poids moléculaire supérieur ou égal à 1.500 et inférieur ou égal à 7.000 ;
- Soit d'un monomère de formule (Ib) :
Si(CH₃)₃-[O-Si(CH₃)₂-]ₘ[O-Si(CH₃)[(CH₂)₃-R]-]ₚO-Si(CH₂)₃ (Ib),
dans laquelle :
R représente un radical monovalent de formule :
-(O-CH₂-CH₂-)ₓ[O-CH₂-CH(CH₃)-]_{y}O-C(=O)-CH=CH₂,
dans lequel x et y représentent, indépendant l'un de l'autre, un nombre entier supérieur ou égal à 0 et inférieur ou égal à 40, étant entendu que la somme x + y est supérieure à 0 et inférieure à 50 ;
m représente un nombre entier supérieur ou égal à 1 et inférieur ou égal à 7, et
p représente indépendamment de m un nombre entier supérieur ou égal à 1 et inférieur à 20 ;
ledit monomères de formule (Ib) ayant un poids moléculaire supérieur ou égal à 1.000 et inférieur ou égal à 4.000 ;
c) d'une proportion massique supérieure ou égale à 1% et inférieure ou égale à 20% d'un monomère de formule (II) :
CH₂=CH(R₁)-C(=O)-O-(CH₂)ₙ-CF₃ (II),
formule (II) dans laquelle le radical R₁ représente un atome d'hydrogène ou un radical méthyle, et n représente un nombre entier égal à 1, 2 ou 3 ;
d) optionnellement d'une proportion massique supérieure à 0% et inférieure ou égale à 5%, d'unités monomériques issues d'un monomère de formule (III) :
R₂-C(=O)-O-[(CH₂-CH(R₄)-O]ₘ-R₃ (III),
formule (III) dans laquelle m représente un nombre supérieur ou égal à 0 et inférieur ou égal à 50, R₂ représente un radical monovalent aliphatique insaturé comprenant de 2 à 4 atomes de carbone, R₄ représente un atome d'hydrogène, un radical méthyle ou un radical éthyle et R₃ représente un radical aliphatique hydrocarboné, linéaire ou ramifié, saturé ou insaturé, comprenant de 8 à 30 atomes de carbone, et
e) optionnellement d'une proportion massique supérieure à 0% et inférieure ou égale à 5% d'au moins un monomère de réticulation diéthylénique ou polyéthylénique.

2. Polyélectrolyte anionique tel que défini à la revendication 1, pour lequel, dans la formule (II), n est égal à 1.

3. Polyélectrolyte anionique tel que défini à l'une quelconque des revendications 1 ou 2, pour lequel, dans la formule (II), R₁ représente un radical méthyle.

4. Polyélectrolyte anionique tel que défini à l'une quelconque des revendications 1 à 3, pour lequel ledit monomère comportant une fonction acide fort libre partiellement ou totalement salifiée, est l'acide 2-méthyl 2-[(1-oxo 2-propènyl) amino] 1-propanesulfonique libre, partiellement salifié ou totalement salifié sous forme de sel de sodium de sel de potassium ou sous forme de sel d'ammonium.

5. Polyélectrolyte anionique tel que défini à l'une quelconque des revendications 1 à 4, pour lequel, ledit monomère de formule (III), est choisi parmi le méthacrylate de béhènyle pentacosaéthoxylé, l'acrylate de lauryle tétraéthoxylé, le méthacrylate de stéaryle eicosaéthoxylé, le méthacrylate de lauryle tétraéthoxylé, ou le méthacrylate de stéaryle.

6. Polyélectrolyte anionique tel que défini à l'une quelconque des revendications 1 à 5, issu de la polymérisation pour 100% massique :
a) d'une proportion massique supérieure ou égale à 75% et inférieure ou égale à 90% d'unités monomériques issues d'un monomère comportant une fonction acide fort, libre, partiellement ou totalement salifiée ;
b) d'une proportion massique supérieure ou égale à 1% et inférieure ou égale à 10% d'unités monomériques issues d'un monomère de formule (la) ou d'un monomère de formule (Ib) ;
c) d'une proportion massique supérieure ou égale à 5% et inférieure ou égale à 20% d'unités monomériques issues du méthacrylate de 2,2,2-trifluoro éthyle ; et
d) optionnellement d'une proportion massique supérieure à 0% et inférieure ou égale à 5% d'unités monomériques issues du composé de formule (III) telle que définie précédemment.

7. Polyélectrolyte anionique tel que défini à l'une quelconque des revendications 1 à 6, **caractérisé en ce qu'**il est réticulé.

8. Polyélectrolyte anionique tel que défini à la revendication 7, **caractérisé en ce que** ledit au moins un monomère de réticulation diéthylénique ou poly-éthylénique est choisi parmi le méthylène bis(acrylamide) ou le triacrylate de triméthylol propane.

9. Polyélectrolyte tel que défini à l'une quelconque des revendications 7 ou 8 issu de la polymérisation pour 100% massique :
a) d'une proportion massique supérieure ou égale à 75% et inférieure ou égale à 85% d'unités monomériques issues d'un monomère comportant une fonction acide fort, libre, partiellement ou totalement salifiée,
b) d'une proportion massique supérieure ou égale à 1% et inférieure ou égale à 10% d'unités monomériques issues d'un monomère de formule (la) ou d'un monomère de formule (Ib) ;
c) d'une proportion massique supérieure ou égale à 5% et inférieure ou égale à 20% d'unités monomériques issues du méthacrylate de 2,2,2-trifluoro éthyle ; et
e) d'une proportion massique supérieure à 0% et inférieure ou égale à 5% massique d'unités monomériques issues d'au moins un monomère de réticulation diéthylénique ou polyéthylénique.

10. Procédé de préparation du polyélectrolyte tel que défini à l'une quelconque des revendications 1 à 9, **caractérisé en ce qu'**il comprend :
Une étape a) de préparation d'un mélange réactionnel comprenant dans les proportions molaires souhaitées et dans un solvant (S), le ou les monomères comportant une fonction acide fort, libre, partiellement ou totalement salifiée, le monomère de formule (la) ou le monomère de formule (Ib) le monomère de formule (II) ; si nécessaire ou si désiré les unités monomériques issues du composé de formule (III) ; et si nécessaire ou si désiré le ou les monomères de réticulation diéthyléniques ou polyéthyléniques, ledit solvant (S) étant :
- Soit une cétone de formule (IV) : 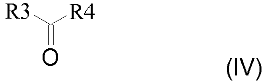 dans laquelle R3 et R4 identiques ou différents, représentent indépendamment l'un de l'autre un radical méthyle, un radical éthyle, un radical isopropyle ou un radical isobutyle ;
- Soit un mélange consistant en, pour 100% molaire :
- de l'eau en une proportion supérieure à 0% molaire et inférieure ou égale à 25% molaire ; et
- une cétone de formule (IV) telle que définie ci-dessus, en une proportion supérieure ou égal à 75% molaire et inférieure à 100% ;
- Soit le tertio-butanol ;
Une étape b) au cours de laquelle la réaction de polymérisation est amorcée par introduction dans ledit mélange réactionnel préparé à l'étape a), d'un initiateur de radicaux libres, puis est laissée se dérouler jusqu'à sa conclusion, pour obtenir un précipité dudit polyélectrolyte, si nécessaire ou si désiré,
une étape c) d'isolation dudit précipité obtenu à l'étape b) par séparation d'avec ledit solvant (S), puis si nécessaire ou si désiré,
une étape d) de séchage dudit précipité résultant de l'étape c).

11. Utilisation du polyélectrolyte anionique tel que défini à l'une quelconque des revendications 1 à 9, comme épaississant et/ou comme stabilisant et/ou comme émulsionnant, d'une composition topique cosmétique, dermopharmaceutique ou pharmaceutique.

12. Composition topique cosmétique **caractérisée en ce qu'**elle comprend de 1% à 5% massique du polyélectrolyte anionique tel que défini à l'une quelconque des revendications 1 à 9, et de 1% massique à 10% massique de glycérol.

13. Composition telle que définie à la revendication 12, **caractérisée en ce qu'**elle est exempte d'huile de silicone.

## Patentansprüche

1. Linearer, verzweigter oder vernetzter anionischer Polyelektrolyt, der aus der Polymerisation für 100 Massenprozent stammt:
a) eines Massenverhältnisses, das größer als oder gleich 70 % und kleiner als oder gleich 98,5 % monomere Einheiten ist, die von einem Monomer stammen, das eine teilweise oder vollständig versalzte freie starke Säurefunktion umfasst;
b) eines Massenverhältnisses, das größer als oder gleich 0,5 % und kleiner als oder gleich 10 % ist,
- entweder eines Monomers mit der Folgenden Formel (Ia) :
R-(CH₂)₃-Si(CH₃)₂-[O-Si(CH₃)₂-]ₙO-Si(CH₃)₂-(CH₂)₃-R (Ia) ,
wobei:
R ein monovalentes Radikal darstellt:
- (O-CH₂-CH₂-)ₓ[O-CH₂-CH(CH₃)-]_{y}O-C (=O) -CH=CH₂,
wobei x und y unabhängig voneinander eine ganze Zahl darstellen, die größer als oder gleich 0 und kleiner als oder gleich 40 ist, vorausgesetzt, dass die Summe x + y größer als 0 und kleiner als 50 ist; und
n eine ganze Zahl darstellt, die größer als oder gleich 0 und kleiner als oder gleich 45 ist, wobei die Monomere mit der Formel (Ia) ein Molekulargewicht aufweisen, das größer als oder gleich 1500 und kleiner als oder gleich 7000 ist;
- oder eines Monomers mit der Formel (Ib):
Si (CH₃)₃-[O-Si(CH₃)₂-]ₘ[O-Si(CH₃)[(CH₂)₃-R]-]ₚO-Si((CH₂)₃ (Ib) ,
wobei:
R ein monovalentes Radikal mit der folgenden Formel darstellt:
-(O-CH₂-CH₂-)[O-CH₂-CH(CH₃)-]_{y}O-C(=O)-CH=CH₂,
wobei x und y unabhängig voneinander eine ganze Zahl darstellen, die größer als oder gleich 0 und kleiner als oder gleich 40 ist, vorausgesetzt, dass die Summe x + y größer als 0 und kleiner als 50 ist;
m eine ganze Zahl darstellt, die größer als oder gleich 1 und kleiner als oder gleich 7 ist, und p unabhängig von m eine ganze Zahl darstellt, die größer als oder gleich 1 und kleiner als 20 ist,
wobei die Monomere mit der Formel (Ib) ein Molekulargewicht aufweisen, das größer als oder gleich 1000 und kleiner als oder gleich 4000 ist;
c) eines Massenverhältnisses, das größer als oder gleich 1 % und kleiner als oder gleich 20 % eines Monomers mit der Formel (II) ist:
CH₂=CH(R₁)-C(=O)-O-(CH₂)ₙ-CF₃ (II),
Formel (II), bei der das Radikal R₁ ein Wasserstoffatom oder ein Methylradikal darstellt und n eine ganze Zahl gleich 1, 2 oder 3 darstellt;
d) optional eines Massenverhältnisses, das größer als 0 % und kleiner als oder gleich 5 % monomere Einheiten ist, die aus einem Monomer mit der folgenden Formel (III) stammen:
R₂-C(=O)-O-(CH₂-CH(R₄)-O]ₘ-R₃ (III),
Formel (III), bei der m eine Zahl darstellt, die größer als oder gleich 0 und kleiner als oder gleich 50 ist, R₂ ein ungesättigtes aliphatisches monovalentes Radikal darstellt, umfassend 2 bis 4 Kohlenstoffatome, R₄ ein Wasserstoffatom, ein Methylradikal oder ein Ethylradikal darstellt und R₃ ein gesättigtes oder ungesättigtes lineares oder verzweigtes aliphatisches Kohlenwasserstoffradikal darstellt, umfassend 8 bis 30 Kohlenstoffatome, und
e) optional eines Massenverhältnisses, das größer als 0 % und kleiner als oder gleich 5% mindestens eines Monomers mit Diethylen- oder Polyethylenvernetzung ist.

2. Anionischer Polyelektrolyt nach Anspruch 1, für den in der Formel (II) n gleich 1 ist.

3. Anionischer Polyelektrolyt nach einem der Ansprüche 1 oder 2, für den in der Formel (II) R₁ ein Methylradikal darstellt.

4. Anionischer Polyelektrolyt nach einem der Ansprüche 1 bis 3, für den das Monomer, das eine teilweise oder vollständig versalzte freie starke Säurefunktion umfasst, 2-Methyl 2-[(1-oxo 2-propenyl) amino] 1-propansulfonsäure ist, teilweise versalzt oder vollständig versalzt in Form eines Natriumsalzes, eines Kaliumsalzes oder in Form eines Ammoniumsalzes.

5. Anionischer Polyelektrolyt nach einem der Ansprüche 1 bis 4, für den das Monomer mit der Formel (III) ausgewählt ist aus pentacosaethoxyliertem Behenylmehtacrylat, tetraethoxyliertem Laurylacrylat, eicosaethoxyliertem Stearylmethacrylat, tetraethoxyliertem Laurylmethacrylat oder Stearylmathacrylat.

6. Anionischer Polyelektrolyt nach einem der Ansprüche 1 bis 5, der aus der Polymerisation für 100 Massenprozent stammt :
a) eines Massenverhältnisses, das größer als oder gleich 75 % und kleiner als oder gleich 90 % monomere Einheiten ist, die von einem Monomer stammen, das eine teilweise oder vollständig versalzte freie starke Säurefunktion umfasst;
b) eines Massenverhältnisses, das größer als oder gleich 1 % und kleiner als oder gleich 10 % monomere Einheiten ist, die aus einem Monomer mit der Formel (Ia) oder einem Monomer mit der Formel (Ib) stammen;
c) eines Massenverhältnisses, das größer als oder gleich 5 % und kleiner als oder gleich 20 % monomere Einheiten ist, die aus 2,2,2-Trifluoroethylmethacrylat stammen; und
d) optional eines Massenverhältnisses, das größer als 0 % und kleiner als oder gleich 5 % monomere Einheiten ist, die aus der Verbindung mit der Formel (III) stammen, wie oben definiert.

7. Anionischer Polyelektrolyt nach einem der Ansprüche 1 bis 6, **dadurch gekennzeichnet, dass** er vernetzt ist.

8. Anionischer Polyelektrolyt nach Anspruch 7, **dadurch gekennzeichnet, dass** das mindestens eine Monomer mit Diethylen- oder Polyethylenvernetzung ausgewählt ist aus Methylenbisacrylamid oder Trimethylolpropan Triacrylat.

9. Anionischer Polyelektrolyt nach einem der Ansprüche 7 oder 8, der aus der Polymerisation für 100 Massenprozent stammt :
a) eines Massenverhältnisses, das größer als oder gleich 75 % und kleiner als oder gleich 85 % monomere Einheiten ist, die von einem Monomer stammen, das eine teilweise oder vollständig versalzte freie starke Säurefunktion umfasst;
b) eines Massenverhältnisses, das größer als oder gleich 1 % und kleiner als oder gleich 10 % monomere Einheiten ist, die aus einem Monomer mit der Formel (Ia) oder einem Monomer mit der Formel (Ib) stammen;
c) eines Massenverhältnisses, das größer als oder gleich 5 % und kleiner als oder gleich 20 % monomere Einheiten ist, die aus 2,2,2-Trifluoroethylmethacrylat stammen; und
e) eines Massenverhältnisses, das größer als 0 % und kleiner als oder gleich 5 % monomere Einheiten ist, die aus mindestens einem Monomer mit Diethylen- oder Polyethylenvernetzung stammen.

10. Verfahren zur Herstellung des Polyelektrolyts nach einem der Ansprüche 1 bis 9, **dadurch gekennzeichnet, dass** es Folgendes umfasst:
einen Schritt a) des Herstellens einer Reaktionsmischung, umfassend in den gewünschten molaren Verhältnissen und in einem Lösemittel (S) das oder die Monomere, umfassend eine teilweise oder vollständig versalzte freie starke Säurefunktion, das Monomer der Formel (Ia) oder das Monomer der Formel (Ib), das Monomer der Formel (II); falls erforderlich oder falls gewünscht die monomeren Einheiten, die aus der Verbindung mit der Formel (III) stammen; und falls erforderlich oder falls gewünscht das oder die Monomere mit Diethylen- oder Polyethylenvernetzung, wobei das Lösemittel (S) Folgendes ist:
- entweder ein Keton mit der Formel (IV) 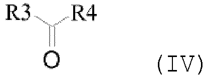 wobei R3 und R4 identisch oder verschieden unabhängig voneinander ein Methylradikal, ein Ethylradikal, ein Isopropylradikal oder ein Isobutylradiakl darstellen;
- oder eine Mischung, die für 100 Molprozent aus Folgendem besteht:
- Wasser in einem Verhältnis, das größer als 0 Molprozent und kleiner als oder gleich 25 Molprozent ist; und
- ein Keton mit der Formel (IV), wie oben definiert, in einem Verhältnis, das größer als oder gleich 75 Molprozent und kleiner als 100 % ist;
- oder tert-Butanol;
einen Schritt b), im Laufe dessen die Polymerisationsreaktion durch Einführen in die Reaktionsmischung, die in Schritt a) hergestellt wurde, eines Initiators mit freien Radikalen gestartet wird, dann bis zu ihrem Ende ablaufen gelassen wird, um eine Ausfällung des Polyelektrolyts zu erhalten, falls erforderlich oder falls gewünscht
einen Schritt c) des Isolierens der Ausfällung, die in Schritt b) erhalten wurde, durch Trennen mit dem Lösemittel (S) dann, falls erforderlich oder falls gewünscht,
einen Schritt d) des Trocknens der Ausfällung, die sich aus Schritt c) ergibt.

11. Verwendung des anionischen Polyelektrolyts nach einem der Ansprüche 1 bis 9 als Verdickungsmittel und/oder als Stabilisator und/oder als Emulgator einer kosmetischen, dermopharmazeutischen oder pharmazeutischen topischen Zusammensetzung.

12. Topische kosmetische Zusammensetzung, **dadurch gekennzeichnet, dass** sie 1 bis 5 Massenprozent des anionischen Polyelektrolyts umfasst, wie in einem der Ansprüche 1 bis 9 definiert, und 1 bis 10 Massenprozent Glycerol.

13. Zusammensetzung nach Anspruch 12, **dadurch gekennzeichnet, dass** sie frei von Silikonöl ist.

## Claims

1. Linear anionic polyelectrolyte, branched or crosslinked, resulting from the 100% by mass polymerisation:
a) of a proportion by mass greater than or equal to 70% and less than or equal to 98.5% of monomeric units issuing from a monomer comprising a strong acid function, partially free or completely salified;
b) of a proportion by mass greater than or equal to 0.5% and less than or equal to 10%:
- either of a monomer of formula (Ia):
R-(CH₂)₃-Si(CH₃)₂-[O-Si(CH₃)₂-]ₙO-Si(CH₃)₂-(CH₂)₃-R (Ia)
in which:
R represents the monovalent radical:
- (O-CH₂-CH₂-)ₓ[O-CH₂-CH(CH₃)-]_{y}O-C(=O)-CH=CH₂,
in which x and y represent, independently of each other, an integer number greater than or equal to 0 and less than or equal to 40, it being understood that the sum x+y is greater than 0 and less than 50; and
n represents an integer number greater than or equal to 0 and less than or equal to 45, said monomers of formula (Ia) having a molecular weight greater than or equal to 1500 and less than or equal to 7000;
- or of a monomer of formula (Ib):
Si(CH₃)₃-[O-Si(CH₃)₂-]ₘ[O-Si(CH₃)[(CH₂)₃-R]-]ₚO-Si(CH₂)₃ (Ib),
in which:
R represents a monovalent radial of formula:
-(O-CH₂-CH₂-)ₓ[O-CH₂-CH(CH₃)-]_{y}O-C(=O)-CH=CH₂,
in which x and y represent, independently of each other, an integer number greater than or equal to 0 and less than or equal to 40, it being understood that the sum x+y is greater than 0 and less than 50;
m represents an integer number greater or equal to 1 and less than or equal to 7, and
p represents independently of m an integer number greater than or equal to 1 and less than 20;
said monomers of formula (Ib) having a molecular weight greater than or equal to 1000 and less than or equal to 4000;
c) a proportion by mass greater than or equal to 1% and less than or equal to 20% of monomer of formula (II):
CH₂=CH(R₁)-C(=O)-O-(CH₂)ₙ-CF₃ (II),
formula (II) in which the radical R₁ represents a hydrogen atom or a methyl radical, and n represents an integer number equal to 1, 2 or 3;
d) optionally a proportion by mass greater than 0% and less than or equal to 5%, of monomeric units issuing from a monomer of formula (III):
R₂-C(=O)-O-(CH₂-CH(R₄)-O]ₘ-R₃ (III),
formula (III) in which m represents a number greater than or equal to 0 and less than or equal to 50, R₂ represents an unsaturated aliphatic monovalent radical, comprising 2 to 4 carbon atoms, R₄ represents a hydrogen atom, a methyl radical or an ethyl radial and R₃ represents a hydrocarbon aliphatic radical, linear or branched, saturated or unsaturated, comprising 8 to 30 carbon atoms, and
e) optionally a proportion by mass greater than 0% and less than or equal to 5% of at least one diethylene or polyethylene crosslinking monomer.

2. Anionic polyelectrolyte as defined in claim 1, for which, in formula (II), n is equal to 1.

3. Anionic polyelectrolyte as defined in either one of claims 1 or 2, for which, in formula (II), R₁ represents a methyl radical.

4. Anionic polyelectrolyte as defined in any one of claims 1 to 3, for which said monomer comprising a partially or completely salified free strong acid function is free 2-methyl 2-[(1-oxo 2-propenyl) amino] 1-propanesulfonic acid, partially salified or completely salified in the form of a sodium salt, a potassium salt or an ammonium salt.

5. Anionic polyelectrolyte as defined in any one of claims 1 to 4, for which said monomer of formula (III) is chosen from pentacosaethoxylated behenyl methacrylate, tetraethoxylated lauryl acrylate, eicosaethoxylated stearyl methacrylate, tetraethoxylated lauryl methacrylate or stearyl methacrylate.

6. Anionic polyelectrolyte as defined in any one of claims 1 to 5, issuing from the 100%-by-mass polymerisation:
a) of a proportion by mass greater than or equal to 75% and less than or equal to 90% of monomeric units issuing from a monomer comprising a free strong acid function, partially or completely salified;
b) of a proportion by mass greater than or equal to 1% and less than or equal to 10% of monomeric units issuing from a monomer of formula (Ia) or a monomer of formula (Ib);
c) of a proportion by mass greater than or equal to 5% and less than or equal to 20% of monomeric units issuing from 2,2,2-trifluoroethyl methacrylate; and
d) optionally a proportion by mass greater than 0% and less than or equal to 5% of monomeric units issuing from the compound of formula (III) as defined previously.

7. Anionic polyelectrolyte as defined in any one of claims 1 to 6, **characterised in that** it is crosslinked.

8. Anionic polyelectrolyte as defined in claim 7, **characterised in that** said at least one diethylene or polyethylene crosslinking monomer is chosen from methylene bis(acrylamide) or trimethylol propane triacrylate.

9. Polyelectrolyte as defined in either one of claims 7 or 8, issuing from the 100%-by-mass polymerisation:
a) of a proportion by mass greater than or equal to 75% and less than or equal to 85% of monomeric units issuing from a monomer comprising a free strong acid function, partially or completely salified;
b) of a proportion by mass greater than or equal to 1% and less than or equal to 10% of monomeric units issuing from a monomer of formula (Ia) or a monomer of formula (Ib);
c) a proportion by mass greater than or equal to 5% and less than or equal to 20% of monomeric units issuing from 2,2,2-trifluoroethyl methacrylate; and
e) of a proportion by mass greater than 0% and less than or equal to 5% by mass of monomeric units issuing from at least one diethylene or polyethylene crosslinking monomer.

10. Method for preparing polyelectrolyte as defined in any one of claims 1 to 9, **characterised in that** it comprises:
A step a) of preparing a reaction mixture comprising, in the required molar proportions and in a solvent (S), the monomer or monomers comprising a free strong acid function, partially or completely salified, the monomer of formula (Ia) or the monomer of formula (Ib) the monomer of formula (II); if necessary or if desired the monomeric units issuing from the compound of formula (III); and, if necessary or if desired, the diethylene or polyethylene crosslinking monomer or monomers, said solvent (S) being:
- either a ketone of formula (IV): 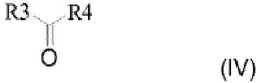 in which R3 and R4, identical or different, represent independently of each other a methyl radical, an ethyl radical, an isopropyl radical or an isobutyl radical;
- or a mixture consisting of, to the extent of 100% molar:
-- water in a proportion greater than 0% molar and less than or equal to 25% molar; and
-- a ketone of formula (IV) as defined above, in a proportion greater than or equal to 75% molar and less than 100%;
- or tert-butanol;
a step b) during which the polymerisation reaction is initiated by introducing, into said reaction mixture prepared at step a), a free-radical initiator, and is then left to take place until it is concluded, in order
to obtain a precipitator of said polyelectrolyte, if necessary or if desired,
a step c) of isolating said precipitate obtained at step b) by separation from said solvent (S), and then,
if necessary or if desired,
a step d) of drying said precipitate resulting from step c) .

11. Use of the anionic polyelectrolyte as defined in any one of claims 1 to 9, as a thickener and/or as a stabiliser and/or as an emulsifier, or a cosmetic, dermopharmaceutical or pharmaceutical topical composition.

12. Cosmetic topical composition **characterised in that** it comprises from 1% to 5% by mass of the anionic polyelectrolyte as defined in any one of claims 1 to 9, and from 1% by mass to 10% by mass of glycerol.

13. Composition as defined in claim 12, **characterised in that** it is free from silicone oil.
